(19) Europäisches Patentamt
European Patent Office
Office européen des brevets

(11) Veröffentlichungsnummer: **0 008 428**
**B1**

(12) # EUROPÄISCHE PATENTSCHRIFT

(45) Veröffentlichungstag der Patentschrift: **31.10.84**

(51) Int. Cl.³: **G 01 N 33/52**, C 09 B 43/18, C 09 B 43/20

(21) Anmeldenummer: **79102920.0**

(22) Anmeldetag: **11.08.79**

(54) **Diagnostisches Mittel zum Nachweis von Leukozyten in Körperflüssigkeiten sowie als Chromogene hierfür geeignete Azofarbstoff-Ester, Verfahren zu deren Herstellung sowie deren Verwendung zur Herstellung diagnostischer Mittel zum Nachweis von Leukozyten in Körperflüssigkeiten.**

(30) Priorität: **22.08.78 DE 2836644**

(43) Veröffentlichungstag der Anmeldung: **05.03.80 Patentblatt 80/5**

(45) Bekanntmachung des Hinweises auf die Patenterteilung: **31.10.84 Patentblatt 84/44**

(84) Benannte Vertragsstaaten: **AT BE CH DE FR GB IT LU NL SE**

(56) Entgegenhaltungen:
**DE - A - 2 065 465**
**FR - A - 2 083 840**
**FR - A - 2 145 664**
**GB - A - 1 167 176**
**JP - A - 45 039 511**
**JP - A - 45 040 188**
**US - A - 2 170 262**
**US - A - 2 870 137**
**US - A - 3 068 072**
**US - A - 3 190 876**

**CHEMICAL ABSTRACTS, Band 71, Nr. 12, 22. September 1969, Seite 68, Zusammenfassung Nr. 51214w, Columbus, Ohio, US, IDA TADAO et al.: "Polymeric dyes. I. Macromolecularization of food dyes"**
**CHEMICAL ABSTRACTS, Band 76, Nr. 5, 31. Januar**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Berger, Dieter, Dr., Bensheimer Strasse 45, D-6806 Viernheim (DE)**
Erfinder: **Braun, Franz, Dr., Ringstrasse 14, D-6149 Rimbach/Odw. (DE)**
Erfinder: **Frey, Günter, Heinigstrasse 45, D-6700 Ludwigshafen (DE)**
Erfinder: **Güthlein, Werner, Dr., Im Senntelch 31, D-6800 Mannheim-Neckerau (DE)**
Erfinder: **Kuhr, Manfred, Dr., Werthmannweg 23, D-6800 Mannheim-31 (Waldhof) (DE)**

(56) Entgegenhaltungen: (Fortsetzung)
**1972, Seite 128, Zusammenfassung Nr. 22256j, Columbus, Ohio, US, J. RUDENS et al.: "Cytochemical detection of napthol AS-D chloroacetate esterase"**
**CHEMICAL ABSTRACTS, Band 89, Nr. 1, 3. Juli 1978, Seite 200, Zusammenfassung Nr. 2110x, Columbus, Ohio, US, G.W. OSBALDISTON et al.: "Cytochemical demonstration of esterases in peripheral blood leukocytes"**
**Klin. W. Schr. 46 Seiten 642-640**

**Die Akte enthält technische Angaben, die nach dem Eingang der Anmeldung eingereicht wurden und die nicht in dieser Patentschrift enthalten sind.**

EP 0 008 428 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Der Nachweis von Leukozyten in Körperflüssigkeiten, insbesondere im Urin, nimmt eine hervorragende Stelle in der Diagnostik der Erkrankungen der Nieren und des Urogenitaltraktes ein.

Bisher wird dieser Nachweis durch mühsames Auszählen der Leukozyten im nicht zentrifugierten Harn oder im Harnsediment geführt. Beiden Methoden ist naturgemäß gemeinsam, daß nur intakte Leukozyten erfaßt werden. Andererseits ist bekannt, daß die Geschwindigkeit der Leukozyten-Lyse je nach Harnmilieu enormen Schwankungen unterworfen ist; so ist z. B. in stark alkalischen Harnen mit einer Leukozyten-Halbwertszeit von nur 60 Minuten zu rechnen. Zu niedrige Leukozytenzahlen bzw. bei längeren Harnstandzeiten sogar falschnegative Befunde sind die Folge.

Vom Lyse-Fehler abgesehen, liefert die quantitative mikroskopische Bestimmung der Leukozyten im nicht zentrifugierten, homogenisierten Harn in der Zählkammer recht zuverlässige Werte. In der Praxis wird diese Methode jedoch nur selten angewandt, da sie mühevoll, ermüdend und zeitraubend ist und den Einsatz geschulten Personals bedingt.

Die überwiegende Mehrzahl der Leukozytenbestimmungen im Harn werden in der medizinischen Praxis nach der sogenannten Gesichtsfeldmethode im Harnsediment durchgeführt. Hierzu muß zunächst das Untersuchungsgut (Sediment) durch Zentrifugieren gewonnen werden. Dabei werden jedoch auch andere Bestandteile des Harnes angereichert, die — wie z. B. Salze und Epithelzellen — die mikroskopische Auszählung der Leukozyten beträchtlich erschweren können. Schwankender Sedimentgehalt, Inhomogenitäten des Sedimentes sowie womöglich unterschiedliche mikroskopische Vergrößerungen oder unterschiedliche optische Ausstattung der Mikroskope führen dazu, daß die hier übliche Angabe über die Anzahl der Leukozyten pro mikroskopischem Gesichtsfeld mit Fehlern von mehreren hundert Prozent behaftet sein kann.

Aufgabe der vorliegenden Erfindung war es daher, ein diagnostisches Mittel bereitzustellen, mit dem die Leukozyten in Körperflüssigkeiten auf einfache und leicht zu handhabende Weise sowie möglichst schnell und vollständig nachgewiesen werden können.

Als Nachweisprinzip für einen solchen Leukozytentest bietet sich eine enzymatische Reaktion an, da die Leukozyten ein breitgefächertes Enzymspektrum besitzen.

In dem US-Patent 3 087 794 ist bereits ein Leukozytennachweis beschrieben und beansprucht, der über die in den granulozytären Leukozyten vorhandene peroxidatische Aktivität geführt wird. Ein saugfähiger Träger, der mit Wasserstoffperoxid und einem organischen Indikator, beispielsweise o-Tolidin, imprägniert ist, zeigt die Anwesenheit von Leukozyten durch die Bildung eines farbigen Oxidationsproduktes an. Ein solcher Test besitzt jedoch entscheidende Nachteile. Einerseits besitzen peroxidatische Reaktionen ganz allgemein gegenüber reduzierenden Substanzen im Harn, wie z. B. gegenüber Ascorbinsäure, eine erhebliche Störanfälligkeit. Andererseits finden sich in mehreren Literaturstellen (s. z. B. L. Mettler, Med. Welt 23, 399 [1972]) Hinweise auf die Instabilität der Leukozytenperoxidase im Harnmilieu, die zu falschnegativen Befunden Anlaß gibt.

Seit einigen Jahren in der histo- und zytochemischen Enzymologie kolorimetrische Nachweismethoden, die auf der esterolytischen Aktivität der in den zu bestimmenden Systemen vorhandener Enzyme beruhen, ihren festen Platz (vgl. z. B. A.G.E. Pearse, Histochemistry, Theoretical and Applied). Im Prinzip werden dabei farblose oder schwach gefärbte Ester eingesetzt, die durch die enzymatische Spaltung zumeist in eine farblose Säure- und eine ebenfalls farblose +lkohol-(Phenol)-Komponente zerfallen. Letztere wird dann in einer der enzymatischen Verseifung folgenden Reaktion zu farbigen Produkten umgesetzt (z. B. Kupplung mit Diazoniumsalzen oder oxidative Reaktionen).

So beschreiben beispielsweise F. Schmalzl und H. Braunsteiner in Klin. Wschr. 46, 642 (1968) einen spezifischen zytochemischen Leukozytenesterase-Nachweis mit Naphthol-AS-D-chloracetat als Substrat und einem Diazoniumsalz zur Bildung der farbigen Azo-Verbindung.

Für ein diagnostisches Mittel zum schnellen und einfachen Nachweis von Leukozyten in Körperflüssigkeiten, wie z. B. im Harn, erweisen sich Zwei-Komponenten-Systeme dieser Art als nicht geeignet, da bekanntlich viele im Harn vorkommende Verbindungen, wie Urobilinogen, Stercobilinogen, Bilirubin u. a., mit Diazoniumsalzen reagieren. Darüber hinaus ist dieser Nachweis viel zu unempfindlich. Beispielsweise zeigen Proben mit 5000 Leukozyten/µl keine Reaktion.

Weiterhin sind Azo-Farbstoffester bekannt, die als Säurerest eine polymerisierbare Gruppe (Acryl- oder Methacrylsäure) enthalten (Ida et al, Chem. Abstr. 71 (1969), 51 214w und US-A-3 190 876) und sich deshalb mit anderen polymerisierbaren Substanzen zu farbechten synthetischen Fasern verarbeiten lassen.

Aus FR-A-2 145 664, GB-A-1 167 176 und US-A-2 170 262 sind Azo-Farbstoffester bekannt, deren Säurerest eine oder mehrere wasserlöslich machende Gruppen enthält, um diese Stoffe zum Drucken oder Färben aus wäßrigen Lösungen verwenden zu können. Gegebenenfalls sind diese Gruppen alkalisch abspaltbar, wodurch der Farbstoff permanent auf das zu färbende Substrat fixiert wird. Umgekehrt sind aus US-A-2 870 137, CH-A-523 958, JP-A-4 539 511 und JP-A-4 540 188 Azo-Farbstoffe bekannt, in denen löslichmachende Hydroxy- oder Aminogruppen durch Acylierung blockiert sind, so daß wasserunlösliche Farbstoffe entstehen, die zum Färben von hydrophoben synthetischen Fasern geeignet sind. Aus der FR-A-2 083 840 und DE-A-2 065 465 sind p,p'-disubstituierte Azobenzole bekannt bei denen einer der Substituenten eine Alkoxy- oder Alkanoyl-Gruppe und der andere eine

Alkylgruppe ist und die auf Grund ihrer linearen Struktur nematische Eigenschaften besitzen.

Allen diesen vorerwähnten Substanzen ist gemeinsam, daß die Estergruppierung durch die in Leukozyten vorkommenden Esterasen und Proteasen nur langsam oder gar nicht gespalten werden, weil die jeweiligen Säurereste nur geringe Affinität zu den Enzymen besitzen. Zur Herstellung diagnostischer Mittel zum Nachweis von Leukozyten sind sie deshalb nicht geeignet.

Überraschenderweise wurde nun gefunden, daß man stabile und schnell anzeigende diagnostische Mittel, mit denen Leukozyten gut in Körperflüssigkeiten nachzuweisen sind, erhält, wenn als Substrate zum Nachweis der in den neutrophilen Leukozyten-Granulozyten vorkommenden Esterasen Azo-Farbstoff-Ester natürlich vorkommender L-$\alpha$-Aminosäuren und entsprechende Di-bis-Pentapeptide verwendet werden.

Gegenstand der vorliegenden Erfindung ist daher ein diagnostisches Mittel zum Nachweis von Leukozyten in Körperflüssigkeiten, bestehend aus einem saugfähigen Träger, der mit einem Chromogen und üblichen Zusatzstoffen imprägniert ist, dadurch gekennzeichnet, daß als Substrat zum Nachweis für die in den Leukozyten vorkommenden Esterasen Azo-Farbstoff-Ester der allgemeinen Formel I

$$A-N=N-B(OR)_n \qquad (I)$$

in der

A einen fünf- oder sechsgliedrigen, gegebenenfalls benzoanellierten Rest mit ein bis zwei Heteroatomen aus der Gruppe N, S, O, der gegebenenfalls ein- oder mehrfach durch F, Cl, Br, niedere Alkylgruppe mit 1—5 C-Atomen und/oder niedere Alkoxygruppe, mit 1—5 C-Atomen, substituiert sein kann oder einen ein- bis dreifach durch niedere Alkylgruppe mit 1—5 C-Atomen, niedere Alkoxygruppe mit 1—5 C-Atomen, Nitro-, Sulfonato- und/oder Benzoylaminogruppen substituierten Phenylrest,

B einen gegebenenfalls ein- bis zweifach durch Sulfonato-, niedere Alkoxygruppe mit 1—5 C-Atomen, und/oder niedere Alkoxy-poly-ethylenoxy-Gruppe, wobei die Alkoxygruppe 1—5 C-Atome besitzt und 1—5 Ethylenoxygruppen enthalten sein können, substituierten Benzol-, Naphthalin- oder Chinolin-Rest,

R einen mit einer in der Peptidchemie üblichen Stickstoffschutzgruppe versehenen Aminosäure- oder Di- bis Pentapeptid-Rest, wobei sowohl die Aminosäure als auch die Aminosäuren des Peptid-Restes natürlich vorkommende L-$\alpha$-Aminosäuren sind und

n die Zahl 1 oder 2 bedeuten,
und die Azogruppe in o- oder p-Stellung zu einer der Gruppen —O—R steht,
eingesetzt werden.

Ein weiterer Gegenstand der vorliegenden Erfindung ist die Verwendung von Azo—Farbstoff-Estern der allgemeinen Formel I zur Herstellung von diagnostischen Mitteln zum Nachweis von Leukozyten in Körperflüssigkeiten.

Sämtliche Azo-Farbstoffe-Ester der allgemeinen Formel I sind neue Verbindungen.

Gegenstand der vorliegenden Erfindung sind daher ferner die Azo-Farbstoff-Ester der allgemeinen Formel I sowie Verfahren zu deren Herstellung.

Die Herstellung der neuen Azo-Farbstoff-Ester der allgemeinen Formel I kann nach an sich bekannten Methoden zur Synthese von Phenolestern erfolgen. Vorzugsweise werden in an sich bekannter Weise die entsprechenden Azo-Farbstoffe der allgemeinen Formel II

$$A-N=N-B(OH)_n \qquad (II)$$

in der A, B und n die oben angegebene Bedeutung haben,
mit Säuren der allgemeinen Formel III

$$HO-R \qquad (III)$$

in der R die oben angegebene Bedeutung hat,
beziehungsweise mit geeigneten reaktiven Derivaten davon umgesetzt.

Zur Herstellung der Aminosäure- und Peptid-Ester werden die in der Peptidchemie üblichen Synthesemethoden angewendet.

Die Azo-Farbstoffe der allgemeinen Formel II sind bekannte Substanzen (vgl. beispielsweise H. R. Hovind, The Analyst, 100, 769 (1975)) oder können in Analogie zu bekannten Verbindungen hergestellt werden.

Unter Fluor, Chlor und Brom in der Definition von A ist Brom bevorzugt.

Die »niedere Alkylgruppe« in der Definition von A sowie die »niedere Alkoxygruppe« von A und B enthalten 1 bis 5, vorzugsweise 1 bis 3 Kohlenstoffatome, wobei die Methyl-, Methoxy- und Ethoxygruppe ganz besonders bevorzugt sind.

Der »polyethylenoxy-Rest« der »niederen Alkoxy-poly-ethylenoxy-Gruppe« in der Definition von B

kann 1 bis 5, vorzugsweise 1 bis 3, gegebenenfalls alkylsubstituierte, Ethylenoxy-Gruppen enthalten. Als »niedere Alkoxy-polyethylenoxy-Gruppe« ist ganz besonders bevorzugt der 3,6-Di-oxaheptyloxy-Rest.

Unter einer »Sulfonatogruppe« in der Definition von A und B sollen sowohl der Sulfonsäurerest selbst, als auch dessen Metallsalze, vorzugsweise die Erdalkali- und Alkalisalze, verstanden werden, wobei das Natriumsalz besonders bevorzugt ist.

Aminosäurereste des Substituenten R sind die Reste der natürlichen L-$\alpha$-Aminosäuren, insbesondere bevorzugt sind Glycin, L-Alanin und L-Phenylalanin.

Unter einem Peptidrest in der Definition des Substituenten R sind Di-, Tri-, Tetra- und Pentapeptide, vorzugsweise Di- und Tripeptide zu verstehen, wobei als Aminosäure-Komponenten die oben erwähnten L-$\alpha$-Aminosäuren Verwendung finden.

Die Verknüpfung der OR-Seitenketten mit den Benzol-, Naphthalin- bzw. Chinolin-Ringen kann in jeder beliebigen Stellung erfolgen. Die Azo-Gruppen sollen jedoch stets in ortho- und/oder para-Stellung zu den OR-Substituenten stehen.

Die erfindungsgemäß als Chromogene verwendeten Azo-Farbstoff-Ester der allgemeinen Formel I werden in Konzentrationen von $10^{-4}$ bis $10^{-1}$ mol/Liter, vorzugsweise $10^{-3}$ bis $10^{-2}$ mol/Liter Imprägnierlösungen eingesetzt.

Ein weiterer Bestandteil des diagnostischen Mittels für den Leukozytennachweis ist ein geeignetes Puffersystem. Hierzu kommen z. B. Phosphat-, Barbiturat-, Borat-, Tris-(hydroxymethyl)-aminomethan-(Tris-), 2-Amino-2-methyl-propandiol-1,3- (Amediol-) oder Aminosäure-Puffer in Frage, wobei pH-Wert und Kapazität so gewählt werden müssen, daß sich nach dem Eintauchen des Teststreifens in die Körperflüssigkeit auf diesem ein pH-Wert von 6—10, vorzugsweise von 7—9, einstellt.

Weiterhin ist es vorteilhaft, bei der Herstellung der erfindungsgemäßen diagnostischen Mittel zum Nachweis von Leukozyten in Körperflüssigkeiten zusätzlich Tenside zu verwenden, da hierdurch kürzere Reaktionszeiten erreicht werden können. Vorzugsweise werden kationenaktive Netzmittel, wie z. B. quaternäre Ammonium-, Pyridinium- oder Imidazoliumsalze in Konzentrationen von 0,05—2%, vorzugsweise 0,1—0,5%, eingesetzt.

Ein weiterer Bestandteil des diagnostischen Mittels für den Leukozytennachweis kann ein geeigneter Komplexbildner sein. Vorzugsweise werden Metallsalze, beispielsweise der Elemente Eisen, Kupfer, Chrom, Kobalt, Nickel, Mangan und Zink, verwendet, die mit den durch Einwirkung der Leukozytenesterasen auf Azo-Farbstoff-Estern der allgemeinen Formel I entstandenen o-Hydroxy-Azo-Farbstoffen unter Farbvertiefung zu entsprechenden Metallchelatkomplexen reagieren. Hierdurch werden kürzere Reaktionszeiten und niedrigere Nachweisgrenzen erreicht. Die Komplexbildner werden in Konzentrationen von $10^{-4}$ bis $10^{-1}$ mol/Liter, vorzugsweise $10^{-3}$ bis $10^{-2}$ mol/Liter Imprägnierlösung eingesetzt.

Zur Herstellung des erfindungsgemäßen diagnostischen Mittels werden vorzugsweise saugfähige Träger, wie z. B. Filterpapier, Cellulose oder Kunstfaservliese, mit Lösungen der erforderlichen, üblicherweise zur Herstellung von Teststreifen verwendeten Reagenzien (Substrat, Puffer, gegebenenfalls Tenside, Komplexbildner usw.) in leichtflüchtigen Lösungsmitteln, wie z. B. Wasser, Methanol, Ethanol oder Aceton, imprägniert. Dies geschieht zweckmäßig in zwei getrennten Schritten: Zunächst wird mit einer wäßrigen Lösung imprägniert, die den Puffer und andere wasserlösliche Zusatzstoffe enthält. Danach wird mit einer Lösung der Esterase-Substrate der allgemeinen Formel I imprägniert. In speziellen Fällen kann auch die umgekehrte Imprägnierfolge angewandt werden. Die fertigen Testpapiere können als solche verwendet werden oder in an sich bekannter Weise an Griffen angeklebt oder vorzugsweise zwischen Kunststoffen und feinmaschigen Netzwerken gemäß DBP 2 118 455 eingesiegelt werden.

Man erhält diagnostische Mittel, die nach Eintauchen in die zu untersuchende Körperflüssigkeit rasch und in einfach zu handhabender Weise die Anwesenheit von Leukozyten über einen Farbumschlag anzeigen. Da die Aktivität der in den neutrophilen Leukozyten-Granulozyten vorkommenden Esterasen auch nach der Lyse der Leukozyten voll erhalten bleibt, werden mit dem erfindungsgemäßen diagnostischen Mittel sowohl intakte als auch lysierte Leukozyten erfaßt. Ein Lysefehler tritt folglich nicht auf.

Beispiel 1

Filterpapier (z. B. Schleicher + Schüll 23 SL) wird nacheinander mit den folgenden Lösungen imprägniert und dann bei 60° C getrocknet.

Lösung 1

| | |
|---|---|
| Tris-(hydroxymethyl)-aminomethan | 0,61 g |
| Wasser, dest. | ca. 30 ml |
| Lösung mit 0,1 N Salzsäure auf einen pH-Wert von 7,0 einstellen | |
| Wasser, dest., ad | 100,0 ml |

Lösung 2

| | |
|---|---|
| Thiazol-2-azo-1'-[2'-(N-benzyloxycarbonyl-L-alanyloxy)-naphthalin] | 46,1 mg |
| Aceton, ad. | 100,0 ml |

Man erhält ein schwach rosa gefärbtes Testpapier, welches sich beim Eintauchen in leukozytenhaltige Harne deutlich rot verfärbt. Es lassen sich nachweisen:

10 000 Leukozyten/µl Harn in ca. 1 Minute
5 000 Leukozyten/µl Harn in ca. 2 Minuten
2 000 Leukozyten/µl Harn in ca. 5 Minuten

Die Empfindlichkeit des Testes liegt bei etwa 2000 Leukozyten/µl.

Testpapiere mit ähnlichen Eigenschaften (Empfindlichkeiten: 2000—10 000 Leukozyten/µl) erhält man, wenn man anstelle von Thiazol-2-azo-1'-[2'-(N-benzyloxycarbonyl-L-alanyloxy)-naphthalin] die folgenden Substrate einsetzt, wobei sich die angegebenen Farbumschläge der Testpapiere beim Eintauchen in leukozytenhaltige Harne beobachten lassen.

1.1. Thiazol-2-azo-2'-[1'-(N-benzyloxycarbonyl-L-alanyloxy)-4'-methoxy-benzol], Farbumschlag: schwach rosa nach violett.
1.2. Thiazol-2-azo-4'-[1',3'-di-(N-benzyloxycarbonyl-L-alanyloxy)-benzol], Farbumschlag: schwach rosa nach rot-violett.
1.3. Thiazol-2-azo-1'-[2'-(N-tert.-butyloxycarbonyl-L-alanyloxy)-naphthalin], Farbumschlag: schwach rosa nach rot.
1.4. Thiazol-2-azo-2'-[1'-(N-benzyloxycarbonyl-L-alanyloxy)-naphthalin], Farbumschlag: rosa nach rot-violett.
1.5. Thiazol-2-azo-1'-[2'-(N-benzyloxycarbonyl-L-alanyloxy)-7'-natriumsulfonato-naphthalin]-di-hydrat, Farbumschlag: rosa nach rot.
1.6. 5-Brom-thiazol-2-azo-1'-[2'-(N-benzyloxycarbonyl-L-alanyloxy)-naphthalin], Farbumschlag: schwach rosa nach rot.
1.7. Benzothiazol-2-azo-1'-[2'-(N-benzyloxycarbonyl-L-alanyloxy)-naphthalin], Farbumschlag: rosa nach rot.
1.8. Benzothiazol-2-azo-2'-[1'-(N-benzyloxycarbonyl-L-alanyloxy)-naphthalin], Farbumschlag: schwach rosa nach rot.
1.9. 6-Methoxy-benzothiazol-2-azo-2'-[1'-benzyloxycarbonyl-L-alanyloxy)-naphthalin], Farbumschlag: rosa nach rot.
1.10. Pyridin-2-azo-4'-[1',3'-di-(N-benzyloxycarbonyl-L-alanyloxy)-benzol], Farbumschlag: beige nach rot-violett.
1.11. Pyridin-2-azo-1'-[2'-(N-benzyloxycarbonyl-L-alanyloxy)-naphthalin], Farbumschlag: beige nach rot.
1.12. Thiazol-2-azo-1'-[2'-(N-benzyloxycarbonyl-glycyloxy)-naphthalin], Farbumschlag: schwach rosa nach rot.
1.13. Thiazol-2-azo-1'-[2'-(N-benzyloxycarbonyl-L-phenylalanyloxy)-naphthalin], Farbumschlag: schwach rosa nach rot.

## Beispiel 2

Filterpapier (z. B. Schleicher + Schüll 23 SL) wird nacheinander mit den folgenden Lösungen imprägniert und dann bei 60° C getrocknet.

### Lösung 1

| | |
|---|---|
| Tris-(hydroxymethyl)-aminomethan | 0,61 g |
| Wasser, dest. | ca. 30 ml |
| Lösung mit 0,1 N Salzsäure auf einen pH-Wert von 7,0 einstellen | |
| Wasser, dest., ad | 100,0 ml |

### Lösung 2

| | |
|---|---|
| Thiazol-2-azo-4'-[1'-(N-benzyloxycarbonyl-L-alanyloxy)-naphthalin] | 46,1 mg |
| Aceton, ad | 100,0 ml |

Man erhält ein rosa gefärbtes Testpapier, welches sich beim Eintauchen in leukozytenhaltige Harne deutlich violett verfärbt. Es lassen sich nachweisen:

5000 Leukozyten/µl Harn in ca. 1 Minute
2000 Leukozyten/µl Harn in ca. 3 Minuten
1000 Leukozyten/µl Harn in ca. 6 Minuten
500 Leukozyten/µl Harn in ca. 10 Minuten

Die Empfindlichkeit des Testes liegt bei etwa 500 Leukozyten/µl.

Testpapiere mit ähnlichen Eigenschaften (Empfindlichkeiten: 500—5000 Leukozyten/µl) erhält man, wenn man anstelle von Thiazol-2-azo-4'-[1'-(N-benzyloxycarbonyl-L-alanyloxy)-naphthalin] die folgenden Substrate einsetzt, wobei sich die angegebenen Farbumschläge der Testpapiere beim Eintauchen in leukozytenhaltige Harne beobachten lassen.

2.1. Thiazol-2-azo-5'-[8'-[N-benzyloxycarbonyl-L-alanyloxy)-chinolin], Farbumschlag: rosa nach rot.
2.2. Thiazol-2-azo-4'-[1'-(N-benzyloxycarbonyl-L-alanyloxy)-5'-methoxy-naphthalin], Farbumschlag: rosa nach rot.
2.3. Thiazol-2-azo-4'-[1'-(N-benzyloxycarbonyl-L-alanyloxy)-5'-(3'',6''-di-oxa-heptyloxy)-naphthalin], Farbumschlag: rosa nach rot.
2.4. 5-Brom-thiazol-2-azo-4'-[1'-(N-benzyloxycarbonyl-L-alanyloxy)-naphthalin], Farbumschlag: schwach rosa nach violett.
2.5. Benzothiazol-2-azo-4'-[1'-(N-benzyloxycarbonyl-L-alanyloxy)-naphthalin], Farbumschlag: rosa nach violett.
2.6. 6-Methoxy-benzothiazol-2-azo-4'-[1'-(N-benzyloxycarbonyl-L-alanyloxy)-naphthalin], Farbumschlag: rosa nach violett.
2.7. Thiazol-2-azo-4'-[1'-(N-benzyloxycarbonyl-L-alanyl-L-alanyloxy)-naphthalin], Farbumschlag: schwach rosa nach violett.
2.8. Thiazol-2-azo-4'-[1'-(N-benzyloxycarbonyl-L-alanyl-L-alanyl-L-alanyloxy)-naphthalin], Farbumschlag: rosa nach violett.
2.9. 5-Methyl-thiazol-2-azo-4'-[1'-(N-benzyloxycarbonyl-L-alanyloxy)-naphthalin], Farbumschlag: rosa nach violett.
2.10. 6-Methyl-benzothiazol-2-azo-4'-[1'-(N-benzyloxycarbonyl-L-alanyloxy)-naphthalin], Farbumschlag: rosa nach violett.

Beispiel 3

Filterpapier (z. B. Schleicher + Schüll 23 SL) wird nacheinander mit den folgenden Lösungen imprägniert und dann bei 60° C getrocknet.

Lösung 1

| | |
|---|---|
| Di-natrium-tetraborat-decahydrat | 1,91 g |
| Wasser, dest. | ca. 30 ml |
| Lösung mit 0,1 N Salzsäure auf einen pH-Wert von 8,0 einstellen | |
| Wasser, dest., ad | 100,0 ml |

Lösung 2

| | |
|---|---|
| 2-Methoxy-4-nitro-benzol-azo-4'-[1'-(N-benzyloxycarbonyl-L-alanyloxy)-naphthalin] | 52,9 mg |
| Aceton, ad | 100,0 ml |

Man erhält ein hell-orange gefärbtes Testpapier, welches sich beim Eintauchen in leukozytenhaltige Harne deutlich rot verfärbt. Es lassen sich nachweisen:

5000 Leukozyten/μl Harn in ca. 1 Minute
2000 Leukozyten/μl Harn in ca. 3 Minuten
1000 Leukozyten/μl Harn in ca. 5 Minuten

Die Empfindlichkeit des Testes liegt bei etwa 1000 Leukozyten/μl.

Testpapiere mit ähnlichen Eigenschaften (Empfindlichkeiten: 1000—5000 Leukozyten/μl) erhält man, wenn man anstelle von 2-Methoxy-4-nitro-benzol-azo-4'-[1'-(N-benzyloxycarbonyl-L-alanyloxy)-naphthalin] die folgenden Substrate einsetzt, wobei sich die angegebenen Farbumschläge der Testpapiere beim Eintauchen in leukozytenhaltige Harne beobachten lassen.

3.1. 4-Natriumsulfonato-benzol-azo-4'-[1'-(N-benzyloxycarbonyl-L-alanyloxy)-naphthalin], Farbumschlag: gelb nach rot.

3.2. 4-Nitro-benzol-azo-4'-[1'-(N-benzyloxycarbonyl-L-alanyloxy)-naphthalin], Farbumschlag: gelb nach rot.

3.3. 2,4-Dinitro-benzol-azo-4'-[1'-(N-benzyloxycarbonyl-L-alanyloxy)-benzol], Farbumschlag: gelb nach rot-violett.

3.4. 2,4-Dinitro-benzol-azo-4'-[1'-(N-benzyloxycarbonyl-L-alanyloxy)-naphthalin], Farbumschlag: gelb nach violett.

3.5. 2-Methoxy-4-nitro-benzol-azo-5'-[8'-(N-benzyloxycarbonyl-L-alanyloxy)-chinolin], Farbumschlag: hellorange nach rot-violett.

3.6. 2-Methoxy-4-nitro-benzol-azo-4'-[1'-(N-benzyloxycarbonyl-L-alanyloxy)-5'-methoxy-naphthalin], Farbumschlag: hellorange nach rot.

3.7. 2-Methoxy-4-nitro-benzol-azo-4'-[1'-(N-benzyloxycarbonyl-L-alanyloxy)-5'-(3",6"-di-oxa-heptyloxy)-naphthalin], Farbumschlag: hellorange nach rot.

3.8. 4-Methoxy-2-nitro-benzol-azo-4'-[1'-(N-benzyloxycarbonyl-L-alanyloxy)-naphthalin], Farbumschlag: hellorange nach violett.

3.9. 2,5-Dimethoxy-4-nitro-benzol-azo-4'-[1'-(N-benzyloxycarbonyl-L-alanyloxy)-naphthalin], Farbumschlag: hellorange nach violett.

3.10. 2,5-Dimethoxy-benzol-azo-4'-[1'-(N-benzyloxycarbonyl-L-alanyloxy)-naphthalin], Farbumschlag: hellorange nach rot.

3.11. 2-Methoxy-4-benzoylamino-5-methyl-benzol-azo-4'-[1'-(N-benzyloxycarbonyl-L-alanyloxy)-naphthalin], Farbumschlag: rosa nach rot.

3.12. 2,5-Dimethoxy-4-benzoylamino-benzol-azo-4'-[1'-(N-benzyloxycarbonyl-L-alanyloxy)-naphthalin], Farbumschlag: orange nach rot.

3.13. 2,5-Diethoxy-4-benzoylamino-benzol-azo-4'-[1'-(N-benzyloxycarbonyl-L-alanyloxy)-naphthalin], Farbumschlag: rosa nach rot.

3.14. 2-Methoxy-4-nitro-benzol-azo-4'-[1'-(N-benzyloxycarbonyl-glycyloxy)-naphthalin],
Farbumschlag: hellorange nach rot.

3.15. 2-Methoxy-4-nitro-benzol-azo-4'-[1'-(N-benzyloxycarbonyl-L-phenylalanyloxy)-naphthalin],
Farbumschlag: hellorange nach rot.

3.16. 2-Methoxy-4-nitro-benzol-azo-4'-[1'-(N-p-toluolsulfonyl-L-alanyloxy)-naphthalin],
Farbumschlag: orange nach rot.

3.17. 2-Methoxy-4-nitro-benzol-azo-4'-[1'-(N-p-toluolsulfonyl-L-alanyloxy)-5'-(3'',6''-di-oxa-heptyloxy)-naphthalin],
Farbumschlag: hellorange nach rot.

## Beispiel 4

Filterpapier (z. B. Schleicher + Schüll 23 SL) wird nacheinander mit den folgenden Lösungen imprägniert und dann bei 60° C getrocknet.

### Lösung 1

| | |
|---|---|
| Tris-(hydroxymethyl)-aminomethan | 0,61 g |
| Wasser, dest. | ca. 30 ml |
| Laurylpyridiniumchlorid | 0,2 g |
| Lösung wird mit 0,1 N Salzsäure auf einen pH-Wert von 7,0 eingestellt | |
| Wasser, dest., ad | 100 ml |

### Lösung 2

| | |
|---|---|
| Thiazol-2-azo-4'-[1'-(N-benzyloxycarbonyl-L-alanyloxy)-naphthalin] | 46,1 mg |
| Aceton, ad | 100 ml |

Man erhält ein rosa gefärbtes Testpapier, welches sich beim Eintauchen in leukozytenhaltige Harne deutlich violett verfärbt. Dabei ergeben sich gegenüber der Rezeptur des Beispiels 2 etwa auf die Hälfte verkürzte Reaktionszeiten.

Auch mit den anderen Substraten der Beispiele 1, 2 und 3 werden mit Netzmitteln, wie beispielsweise dem oben verwendeten Laurylpyridiniumchlorid, oder auch z. B. mit Benzyltrimethylammoniumchlorid oder N-Palmityl-N-methyl-benzimidazoliumchlorid, Testpapiere erhalten, die gegenüber analogen Testpapieren ohne Netzmittel etwa auf die Hälfte verkürzte Reaktionszeiten aufweisen.

## Beispiel 5

Filterpapier (z. B. Schleicher + Schüll 23 SL) wird nacheinander mit den folgenden Lösungen imprägniert und dann bei 60° C getrocknet.

### Lösung 1

| | |
|---|---|
| Tris-(hydroxymethyl)-aminomethan | 0,61 g |
| Wasser, dest. | ca. 30 ml, |
| Lösung mit 0,1 N Salzsäure auf einen pH-Wert von 7,0 einstellen | |
| Wasser, dest., ad | 100 ml |

### Lösung 2

| | |
|---|---|
| Thiazol-2-azo-1'-[2'-(N-benzyloxycarbonyl-L-alanyloxy)-naphthalin] | 46,1 mg |
| Zinkacetat-dihydrat | 21,9 mg |
| Aceton, ad | 100 ml |

Man erhält ein schwach rosa gefärbtes Testpapier, welches sich beim Eintauchen in leukozytenhaltige Harne deutlich blau-violett verfärbt. Gegenüber der Rezeptur des Beispiels 1 ergeben sich etwa auf die Hälfte verkürzte Reaktionszeiten, weiterhin verbessert sich die Empfindlichkeit des Testes auf etwa 1000 Leukozyten/µl.

Auch mit den anderen Substraten des Beispiels 1 werden mit Metallsalzen, wie beispielsweise dem oben verwendeten Zinkacetat oder auch z. B. mit Kupfer-II-chlorid oder Eisen-III-chlorid, Testpapiere erhalten, die gegenüber analogen Testpapieren ohne Metallsalze erheblich verkürzte Reaktionszeiten und etwa um den Faktor 2—3 verbesserte Nachweisgrenzen aufweisen.

### Beispiel 6

Thiazol-2-azo-1'-[2'-(N-benzyloxycarbonyl-L-alanyloxy)-naphthalin]

Lösung 1

Zur Herstellung des Säurechlorids nach der Einstufen-Methode werden 6,70 g (0,03 mol) N-Benzyloxycarbonyl-L-alanin in 50 ml abs. Dimethylformamid (DMF) gelöst und auf −30° C abgekühlt. Dann werden unter Rühren und Kühlen 2,4 ml (0,033 mol) Thionylchlorid zupipettiert und das Reaktionsgemisch unter Wasserausschluß im Kältebad bei −30° C belassen.

Lösung 2

3,83 g (0,015 mol) 1-(2'-Thiazolylazo)-2-naphthol (TAN) werden in 75 ml abs. DMF gelöst und auf −30° C abgekühlt.

Umsetzung

Man gießt Lösung 2 zur Lösung 1, fügt als Chlorwasserstoff-Acceptor 4,8 ml (0,034 mol) Triethylamin hinzu und rührt 6 h ohne Kühlung, wobei man die Temperatur in 1,5 h auf 20° C ansteigen läßt. Dann wird die Reaktionslösung wieder auf −30° C gekühlt und die gleiche Menge der frisch hergestellten Säurechloridlösung 1, sowie 4,8 ml Triethylamin zugegeben. Die Temperatur läßt man in 1,5 h wieder auf 20° C steigen und dann das Gemisch 18 h bei Raumtemperatur zu Ende reagieren. Die Umsetzung wird zweckmäßig chromatographisch verfolgt und die Zugabe des erforderlichen, überschüssigen Säurechlorids und Triethylamins dementsprechend vorgenommen.

Zur Aufarbeitung wird das Reaktionsgemisch im Vakuum zur Trockne gebracht (maximal 50° C Badtemperatur). Der Rückstand wird in 100 ml Essigester aufgenommen und nacheinander zweimal mit 30 ml 1 N Zitronensäure, 20 ml Wasser, 50 ml 5—10%iger Natriumhydrogencarbonatlösung und zweimal mit 25 ml Wasser gewaschen. Die Essigesterphase wird nach dem Trocknen mit Natriumsulfat im Vakuum eingeengt. Der Rückstand wird säulenchromatographisch an Kieselgel mit einem Toluol-Dioxan-Gemisch (9:1) gereinigt. Nach dem Abdestillieren des Lösungsmittelgemisches im Vakuum und Anrühren des Rückstandes mit Essigester erhält man 4,9 g (70,5%) Thiazol-2-azo-1'-[2'-(N-benzyloxycarbonyl-L-alanyloxy)-naphthalin], gelbe Kristalle, Schmp. 162° C.

In analoger Weise erhält man aus den entsprechend substituierten Azofarbstoffen und N-geschützten Aminosäuren bzw. Peptiden die folgenden Verbindungen.

6.1. Thiazol-2-azo-2'-[1'-(N-benzyloxycarbonyl-L-alanyloxy)-4'-methoxy-benzol], orangefarbene Kristalle, Schmp. 148—150° C.

6.2. Thiazol-2-azo-4'-[1',3'-di-(N-benzyloxycarbonyl-L-alanyloxy)-benzol], gelb-orangefarbene Kristalle, Schmp. 133—135° C.

6.3. Thiazol-2-azo-2'-[1'-(N-benzyloxycarbonyl-L-alanyloxy)-naphthalin], orangefarbene Kristalle, Schmp. 174—175° C.

6.4. Thiazol-2-azo-4'-[1'-(N-benzyloxycarbonyl-L-alanyloxy)-naphthalin], orangefarbene Kristalle, Schmp. 132° C.

6.5. Thiazol-2-azo-5'-[8'-(N-benzyloxycarbonyl-L-alanyloxy)-chinolin], gelbe Kristalle, Schmp. 134° C.

6.6. Thiazol-2-azo-1'-[2'-(N-benzyloxycarbonyl-L-alanyloxy)-7'-natriumsulfonato-naphthalin]-di-hydrat,
orangefarbenes Pulver,
DC: Fertigplatte Kieselgel,
(Laufmittel: Isopropanol-Essigsäurebutylester-Wasser 5 : 3 : 2,
Detektion: UV, Eigenfarbe,
$R_F$-Wert: 0,49).

6.7. Thiazol-2-azo-4'-[1'-(N-benzyloxycarbonyl-L-alanyloxy)-5'-methoxy-naphthalin], orangefarbene Kristalle, Schmp. 127° C.

6.8. Thiazol-2-azo-4'-[1'-(N-benzyloxycarbonyl-L-alanyloxy)-5'-(3'',6''-di-oxa-heptyl-oxy)-naphthalin], rotbraune, amorphe Substanz, DC: Fertigplatte Kieselgel, (Laufmittel: Chloroform-Methanol 50 : 1, Detektion: UV, Eigenfarbe, $R_F$-Wert: 0,41).

6.9. 5-Brom-thiazol-2-azo-1'-[2'-(N-benzyloxycarbonyl-L-alanyloxy)-naphthalin], orangefarbene Kristalle, Schmp. 192° C.

6.10. 5-Brom-thiazol-2-azo-4'-[1'-(N-benzyloxycarbonyl-L-alanyloxy)-naphthalin], orangefarbene Kristalle, Schmp. 162° C.

6.11. Benzothiazol-2-azo-1'-[2'-(N-benzyloxycarbonyl-L-alanyloxy)-naphthalin], orangefarbene Kristalle, Schmp. 190° C.

6.12. Benzothiazol-2-azo-2'-[1'-(N-benzyloxycarbonyl-L-alanyloxy)-naphthalin], orangefarbene Kristalle, Schmp. 148—150° C.

6.13. Benzothiazol-2-azo-4'-[1'-(N-benzyloxycarbonyl-L-alanyloxy)-naphthalin], orangefarbene Kristalle, Schmp. 180—182° C.

6.14. 6-Methoxy-benzothiazol-2-azo-2'-[1'-(N-benzyloxycarbonyl-L-alanyloxy)-naphthalin], orangefarbene Kristalle, Schmp. 148—150° C.

6.15. 6-Methoxy-benzothiazol-2-azo-4'-[1'-(N-benzyloxycarbonyl-L-alanyloxy)-naphthalin], gelb-orangefarbene Kristalle, Schmp. 194—196° C.

6.16. Pyridin-2-azo-4'-[1',3'-di-(N-benzyloxycarbonyl-L-alanyloxy)-benzol], bräunliches, amorphes Pulver, DC: Fertigplatte Kieselgel, (Laufmittel: Toluol-Dioxan 5 : 1 in Eisessig-Atmosphäre, Detektion: UV, Kupferacetat-Ammoniak, $R_F$-Wert: 0,34).

6.17. Pyridin-2-azo-1'-[2'-(N-benzyloxycarbonyl-L-alanyloxy)-naphthalin], bräunliche Kristalle, Schmp. 117° C.

6.18. 4-Natriumsulfonato-benzol-azo-4'-[1'-(N-benzyloxycarbonyl-L-alanyloxy)-naphthalin], orangefarbene Kristalle, Schmp. 264° C.

6.19. 4-Nitro-benzol-azo-4'-[1'-(N-benzyloxycarbonyl-L-alanyloxy)-naphthalin], rotbraune Kristalle, Schmp. 145° C.

6.20. 2,4-Dinitro-benzol-azo-4'-[1'-(N-benzyloxycarbonyl-L-alanyloxy)-benzol], ockerfarbene Kristalle, Schmp. 110° C.

6.21. 2,4-Dinitro-benzol-azo-4'-[1'-(N-benzyloxycarbonyl-L-alanyloxy)-naphthalin], orangefarbene Kristalle, Schmp. 154° C.

6.22. 2-Methoxy-4-nitro-benzol-azo-4'-[1'-(N-benzyloxycarbonyl-L-alanyloxy)-naphthalin], rotbraune Kristalle, Schmp. 186—187° C.

6.23. 2-Methoxy-4-nitro-benzol-azo-5'-[8'-(N-benzyloxycarbonyl-L-alanyloxy)-chinolin], orangefarbene Kristalle, Schmp. 225—230° C.

6.24. 2-Methoxy-4-nitro-benzol-azo-4'-[1'-(N-benzyloxycarbonyl-L-alanyloxy)-5'-methoxy-naphthalin], orangefarbene Kristalle, Schmp. 165—167° C.

6.25. 2-Methoxy-4-nitro-benzol-azo-4'-[1'-(N-benzyloxycarbonyl-L-alanyloxy)-5'-(3'',6''-di-oxa-heptyloxy)-naphthalin], orangefarbene Kristalle, Schmp. 118—120° C.

6.26. 4-Methoxy-2-nitro-benzol-azo-4'-[1'-(N-benzyloxycarbonyl-L-alanyloxy)-naphthalin], rotbraune Kristalle, Schmp. 140° C.

6.27. 2,5-Dimethoxy-4-nitro-benzol-azo-4'-[1'-(N-benzyloxycarbonyl-L-alanyloxy)-naphthalin], orangefarbene Kristalle, Schmp. 198—201° C.

6.28. 2,5-Dimethoxy-benzol-azo-4'-[1'-(N-benzyloxycarbonyl-L-alanyloxy)-naphthalin], orangefarbene Kristalle, Schmp. 145° C.

6.29. 2-Methoxy-4-benzoylamino-5-methyl-benzol-azo-4'-[1'-(N-benzyloxycarbonyl-L-alanyloxy)-naphthalin], rotbraune Kristalle, Schmp. 128—130° C.

6.30. 2,5-Dimethoxy-4-benzoylamino-benzol-azo-4'-[1'-(N-benzyloxycarbonyl-L-alanyloxy)-naphthalin], orangefarbene Kristalle, Schmp. 141—143° C.

6.31. 2,5-Diethoxy-4-benzoylamino-benzol-azo-4'-[1'-(N-benzyloxycarbonyl-L-alanyloxy)-naphthalin], rotbraune Kristalle, Schmp. 173—174° C.

6.32. Thiazol-2-azo-1'-[2'-(N-benzyloxycarbonyl-L-phenylalanyloxy)-naphthalin], orangefarbene Kristalle, Schmp. 169° C.

6.33. 2-Methoxy-4-nitro-benzol-azo-4'-[1'-(N-benzyloxycarbonyl-L-phenylalanyloxy)-naphthalin], rotbraune Kristalle, Schmp. 205—207° C.

6.34. Thiazol-2-azo-4'-[1'-(N-benzyloxycarbonyl-L-alanyl-L-alanyloxy)-naphthalin], orangefarbene Kristalle, Schmp. 175° C.

6.35. Thiazol-2-azo-4'-[1'-(N-benzyloxycarbonyl-L-alanyl-L-alanyl-L-alanyloxy)-naphthalin], hellorangefarbene Kristalle, Schmp. 201° C.

6.36. 5-Methyl-thiazol-2-azo-4'-[1'-(N-benzyloxycarbonyl-L-alanyloxy)-naphthalin]

6.37. 6-Methyl-benzothiazol-2-azo-4'-[1'-(N-benzyloxycarbonyl-L-alanyloxy)-naphthalin]

6.38. 2-Methoxy-4-nitro-benzol-azo-4'-[1'-(N-p-toluolsulfonyl-L-alanyloxy)-naphthalin], rötliches, amorphes Pulver, DC: Fertigplatte Kieselgel (Laufmittel: Toluol-Dioxan 6 : 1, Detektion: UV, Eigenfarbe, $R_F$-Wert: 0,38).

6.39. 2-Methoxy-4-nitro-benzol-azo-4'-[1'-(N-p-toluolsulfonyl-L-alanyloxy)-5'-(3'',6''-di-oxa-heptyloxy)-naphthalin], orangefarbene Kristalle, Schmp. 61—63° C.

Beispiel 7

Thiazol-2-azo-1'-[2'-(N-tert.-butyloxycarbonyl-L-alanyloxy)-naphthalin]

1,89 g (0,01 mol) N-tert.-Butyloxycarbonyl-L-alanin und 2,55 g (0,01 mol) 1-(2'-Thiazolylazo)-2-naphthol (TAN) werden in 50 ml abs. Pyridin bei Raumtemperatur gelöst und mit der Lösung von 2,2 g (0,0107 mol) Dicyclohexylcarbodiimid (DCC) in 20 ml Pyridin versetzt. Unter Wasserausschluß rührt man ca. 24 h bei Raumtemperatur, dann gibt man noch einmal 1,89 g (0,01 mol) N-tert.-Butyloxycarbonyl-L-alanin und 2,2 g (0,0107 mol) DCC zur Reaktionslösung und rührt weitere 24 h bei Raumtemperatur. Der bereits nach kurzer Zeit sich abscheidende N,N'-Dicyclohexylharnstoff wird abgesaugt, das Lösungsmittel im Vakuum abdestilliert und der Rückstand in 100 ml Essigester aufgenommen. Weiterer sich abscheidender N,N'-Dicyclohexylharnstoff wird abgesaugt und die klare Essigesterlösung nacheinander je zweimal mit 30 ml 1 N Zitronensäure, 20 ml Wasser, 50 ml 5—10%iger Natriumhydrogencarbonatlösung und mit 25 ml Wasser gewaschen. Nach dem Trocknen mit Natriumsulfat wird die Essigesterphase im Vakuum eingedampft. Der klebrige Rückstand wird säulenchromatographisch an einer Kieselgelsäule mit einem Toluol-Dioxan-Gemisch (9 : 1) gereinigt. Man erhält so 2,65 g (62% d. Th.) Thiazol-2-azo-1'-[2'-(N-tert.-butyloxycarbonyl-L-alanyloxy)-naphthalin], orangefarbene Kristalle, Schmp. 142—144° C.

In analoger Weise erhält man aus den entsprechenden Aminosäuren, den entsprechend substituierten Azo-Farbstoffen und DCC die folgenden Verbindungen.

7.1. Thiazol-2-azo-1'-[2'-(N-benzyloxycarbonyl-glycyloxy)-naphthalin], orangefarbene Kristalle, Schmp. 166° C.

7.2. 2-Methoxy-4-nitro-benzol-azo-4'-[1'-(N-benzyloxycarbonyl-glycyloxy)-naphthalin], gelb-orangefarbene Kristalle, Schmp. 185—189° C.

**Patentansprüche**

1. Diagnostisches Mittel zum Nachweis von Leukozyten in Körperflüssigkeiten, bestehend aus einem saugfähigen Träger, der mit einem Chromogen und einer geeigneten Puffersubstanz imprägniert ist, dadurch gekennzeichnet, daß als Chromogene Azo-Farbstoff-Ester der allgemeinen Formel I

$$A-N=N-B(OR)_n \qquad (I)$$

in der

A einen fünf- oder sechsgliedrigen, gegebenenfalls benzoannellierten Rest mit ein bis zwei Heteroatomen aus der Gruppe N, S, O, der gegebenenfalls ein- oder mehrfach durch F, Cl, Br, niedere Alkylgruppe mit 1—5 C-Atomen und/oder niedere Alkoxygruppe, mit 1—5 C-Atomen, substituiert sein kann oder einen ein- bis dreifach durch niedere Alkylgruppe mit 1—5 C-Atomen, niedere Alkoxygruppe mit 1—5 C-Atomen, Nitro-, Sulfonato- und/oder Benzoylaminogruppen substituierten Phenylrest,

B einen gegebenenfalls ein- bis zweifach durch Sulfonato-, niedere Alkoxygruppe mit 1—5 C-Atomen, und/oder niedere Alkoxy-poly-ethylenoxy-Gruppe, wobei die Alkoxygruppe 1—5 C-Atome besitzt und 1—5 Ethylenoxygruppen enthalten sein können, substituierten Benzol-, Naphthalin- oder Chinolin-Rest,

R einen mit einer in der Peptidchemie üblichen Stickstoffschutzgruppe versehenen Aminosäure- oder Di-bis Pentapeptid-Rest wobei sowohl die Aminosäure als auch die Aminsäuren des Peptid-Restes natürlich vorkommende L-$\alpha$-Aminosäuren sind und

n die Zahl 1 oder 2 bedeuten,
und die Azogruppe in o- oder p-Stellung zu einer der Gruppen O—R steht,
eingesetzt werden;
wobei die Puffersubstanz mit der Testflüssigkeit einen pH-Wert von 6—10 einstellt und die Chromogene in einer Konzentration von $10^{-4}$—$10^{-1}$ mol/l enthalten sind.

2. Diagnostisches Mittel gemäß Anspruch 1, dadurch gekennzeichnet, daß der Träger zusätzlich ein Netzmittel und/oder einen Komplexbildner enthält.

3. Verwendung von Azo-Farbstoff-Estern der allgemeinen Formel I

$$A-N=N-B(OR)_n \qquad (I)$$

in der

A einen fünf- oder sechsgliedrigen, gegebenenfalls benzoannelierten Rest mit ein bis zwei Heteroatomen aus der Gruppe N, S, O der gegebenenfalls ein- oder mehrfach durch F, Cl, Br, niedere Alkylgruppe mit 1—5 C-Atomen und/oder niedere Alkoxygruppe mit 1—5 C-Atomen substituiert sein kann oder einen ein- bis dreifach durch niedere Alkylgruppe mit 1—5 C-Atomen, niedere Alkoxygruppe mit 1—5 C-Atomen, Nitro-, Sulfonato- und/oder Benzoylaminogruppen substituierten Phenylrest,

B einen gegebenenfalls ein- bis zweifach durch Sulfonato-, niedere Alkoxygruppe mit 1—5 C-Atomen und/oder niedere Alkoxy-poly-ethylenoxy-Gruppe, wobei die Alkoxygruppe 1—5 C-Atome besitzt und 1—5 Ethylenoxygruppen enthalten sein können, substituierten Benzol-, Naphthalin- oder Chinolin-Rest,

R einen mit einer in der Peptidchemie üblichen Stickstoffschutzgruppe versehenen Aminosäure- oder Di-bis Pentapeptid-Rest wobei sowohl die Aminosäure als auch die Aminosäuren des Peptid-Restes natürlich vorkommende L-$\alpha$-Aminosäuren sind und

n die Zahl 1 oder 2 bedeuten,
und die Azogruppe in o- oder p-Stellung zu einer der Gruppen O—R steht zur Herstellung diagnostischer Mittel zum Nachweis von Leukozyten.

4. Azo-Farbstoff-Ester der allgemeinen Formel I

$$A-N=N-B(OR)_n \qquad (I)$$

in der

A einen fünf- oder sechsgliedrigen, gegebenenfalls benzoannellierten Rest mit ein bis zwei Heteroatomen aus der Gruppe N, S, O, der gegebenenfalls ein- oder mehrfach durch F, Cl, Br, niedere Alkylgruppe mit 1—5 C-Atomen und/oder niedere Alkoxygruppe mit 1—5 C-Atomen substituiert sein kann oder einen ein- bis dreifach durch niedere Alkylgruppe mit 1—5 C-Atomen, niedere Alkoxygruppe mit 1—5 C-Atomen, Nitro-, Sulfonato- und/oder Benzoylaminogruppen substituierten Phenylrest,

B einen gegebenenfalls ein- bis zweifach durch Sulfonato-, niedere Alkoxygruppe mit 1—5 C-Atomen und/oder niedere Alkoxy-poly-ethylenoxy-Gruppe, wobei die Alkoxygruppe 1—5 C-Atome besitzt und 1—5 Ethylenoxygruppen enthalten sein können, substituierten Benzol-, Naphthalin- oder Chinolin-Rest,

R einen mit einer in der Peptidchemie üblichen Stickstoffschutzgruppe versehenen Aminosäure- oder Peptid-Rest wobei sowohl die Aminosäure als auch die Aminosäuren des Peptid-Restes natürlich vorkommende L-$\alpha$-Aminosäuren sind und

n die Zahl 1 oder 2 bedeuten,
und die Azogruppe in o- oder p-Stellung zu einer der Gruppen O—R steht.

5. Verfahren zur Herstellung von Azo-Farbstoff-Estern der allgemeinen Formel I

$$A-N=N-B(OR)_n \quad (I)$$

in der

A einen fünf- oder sechsgliedrigen, gegebenenfalls benzoannellierten Rest mit ein bis zwei Heteroatomen aus der Gruppe N, S, O der gegebenenfalls ein- oder mehrfach durch F, Cl, Br, niedere Alkylgruppen mit 1—5 C-Atomen und/oder niedere Alkoxygruppe mit 1—5 C-Atomen substituiert sein kann oder
einen ein- bis dreifach durch niedere Alkylgruppe mit 1—5 C-Atomen, niedere Alkoxygruppe mit 1—5 C-Atomen, Nitro-, Sulfonato- und/oder Benzoylaminogruppen substituierten Phenylrest,

B einen gegebenenfalls ein- bis zweifach durch Sulfonato-, niedere Alkoxy- und/oder niedere Alkoxy-poly-ethylenoxy-Gruppe, wobei die Alkoxygruppe 1—5 C-Atome besitzt und 1—5 Ethylenoxygruppen enthalten sein können, substituierten Benzol-, Naphthalin- oder Chinolin-Rest,

R einen mit einer in der Peptidchemie üblichen Stickstoffschutzgruppe versehenen Aminosäure- oder Peptid-Rest wobei sowohl die Aminosäure als auch die Aminosäuren des Peptid-Restes natürlich vorkommende L-$\alpha$-Aminosäuren sind und

n die Zahl 1 oder 2 bedeuten,
und die Azogruppe in o- oder p-Stellung zu einer der Gruppen O—R steht dadurch gekennzeichnet, daß man in an sich bekannter Weise Verbindungen der allgemeinen Formel II

$$A-N=N-B(OH)_n \quad (II)$$

in der
A, B und n die oben angegebene Bedeutung haben,
mit Säuren der allgemeinen Formel III

$$HO-R \quad (III)$$

in der
R die oben angegebene Bedeutung hat,
beziehungsweise mit geeigneten reaktiven Derivaten davon umsetzt.

**Claims**

1. Diagnostic agent for the detection of leukocytes in body fluids, consisting of an absorbent carrier which is impregnates with a chromogen and a suitable buffer substance, characterised in that as chromogen there are used azo dyestuff esters of the general formula I

$$A-N=N-B(OR)_n \quad (I)$$

in which

A signifies a five- or six-membered, optionally benzoannellated radical with one to two heteroatoms from the group N, S, O, which can optionally be substituted one or more times by F, Cl, Br, lower alkyl groups with 1—5 C-atoms and/or lower alkoxy groups with 1—5 C-atoms, or a phenyl radical substituted one to three times by lower alkyl groups with 1—5 C-atoms, lower alkoxy groups with 1—5 C-atoms, nitro, sulphonato and/or benzoylamino groups,

B signifies a benzene, naphthalene or quinoline radical optionally substituted one to two times by sulphonato, lower alkyl groups with 1—5 C-atoms and/or lower alkoxy-polyethyleneoxy groups, whereby the alkoxy group possesses 1—5 C-atoms and 1—5 ethyleneoxy groups can be present,

R signifies an amino acid or di- to pentapeptide residue provided with a nitrogen protective group usual in peptide chemistry, whereby not only the amino acid but also the amino acids of the peptide residue are naturally-occurring L-$\alpha$-amino acids and

n signifies the number 1 or 2,
and the azo group is in the o- or p-position to one of the groups O—R;
whereby the buffer substance adjusts with the test liquid a pH value of 6—10 and the chromogens are contained in a concentration of $10^{-4}$—$10^{-1}$ mol/l.

2. Diagnostic agent according to claim 1, characterised in that the carrier additionally contains a wetting agent and/or a complex former.

3. Use of azo dyestuffs of the general formula I

$$A-N=N-B(OR)_n \quad (I)$$

in which

A signifies a five- or six-membered, option-allyl benzo-annellated radical with one to two hetero atoms from the group N, S, O, which can optionally be substituted one or more times by F, Cl, Br, lower alkyl groups with 1—5 C-atoms and/or lower alkoxy groups with 1—5 C-atoms, or a phenyl radical substituted one to three times by lower alkyl groups with 1—5 C-atoms, lower alkoxy groups with 1—5 C-atoms, nitro, sulphonato and/or benzoylamino groups,

B signifies a benzene, naphthalene or quinoline radical optionally substituted once or twice by sulphonato, lower alkoxy groups with 1—5 C-atoms and/or lower alkoxy-polyethyleneoxy groups, whereby the alkoxy group possesses 1—5 C-atoms and 1—5 ethyleneoxy groups can be present,

R signifies an amino acid or di- to pentapeptide residue provided with a nitrogen protective group usual in peptide chemistry, whereby not only the amino acid but also the amino acids of the peptide residue are naturally-occurring L-$\alpha$-amino acids and

n signifies the number 1 or 2,
and the azo group stands in the o- or p-position to one of the groups O—R, for the preparation of diagnostic agents for the detection of leukocytes.

4. Azo dyestuff esters of the general formula I

$$A-N=N-B(OR)_n \quad (I)$$

in which

A signifies a five- or six-membered, optionally benzoannellated radical with one to two hetero atoms from the group N, S, O, which can optionally be substituted one or more times by F, Cl, Br, lower alkyl groups with 1—5 C-atoms and/or lower alkoxy groups with 1—5 C-atoms, or a phenyl radical substituted one to three times by lower alkyl groups with 1—5 C-atoms, lower alkoxy groups with 1—5 C-atoms, nitro, sulphonato and/or benzoylamino groups,

B signifies a benzene, naphthalene or quinoline radical optionally substituted one to two times by sulphonato, lower alkoxy groups with 1—5 C-atoms and/or lower alkoxy-polyethyleneoxy groups, whereby the alkoxy group possesses 1—5 C-atoms and 1—5 ethyleneoxygroups can be present,

R signifies an amino acid or peptide residue provided with a nitrogen protective group usual in peptide chemistry, whereby not only the amino acid but also the amino acids of the peptide residue are naturally-occurring L-$\alpha$-amino acids and

n signifies the number 1 or 2,
and the azo group stands in o- or p-position to one of the O—R groups.

5. Process for the preparation of azo dyestuff esters of the general formula I

$$A-N=N-B(OR)_n \quad (I)$$

in which

A signifies a five- or six-membered optionally benzoannellated radical with one to two hetero atoms of the group N, S, O, which can optionally be substituted one or more times by F, Cl, Br, lower alkyl groups with 1—5 C-atoms and/or lower alkoxy groups with 1—5 C-atoms, or a phenyl radical substituted one to three times by lower alkyl groups with 1—5 C-atoms, lower alkoxy groups with 1—5 C-atoms, nitro, sulphonato and/or benzoylamino groups,

B signifies a benzene, naphthalene or quinoline radical optionally substituted one to two times by sulphonato, lower alkoxy and/or lower alkoxypolyethyleneoxy groups, whereby the alkoxy group possesses 1—5 C-atoms and 1—5 ethyleneoxy groups can be present,

R signifies an amino acid or peptide residue provided with a nitrogen protective group usual in peptide chemistry, whereby not only the amino acid but also the amino acids of the peptide residue are naturally-occurring L-$\alpha$-amino acids and

n signifies the number 1 or 2,
and the azo group stands in o- or p-position to one of the O—R groups,
characterised in that, in per se known manner, one reacts compounds of the general formula II

$$A-N=N-B(OH)_n \qquad (II)$$

in which A, B and n have the above-given meaning, with acids of the general formula III

$$HO-R \qquad (III)$$

in which R has the above-given meaning, or with suitable reactive derivatives thereof.

**Revendications**

1. Agent de diagnostic pour la mise en évidence de leucocytes dans les liquides corporels, comprenant un support absorbant imprégné d'un chromogène et d'une substance tampon appropriée, caractérisé en ce qu'on utilise comme chromogène un ester de colorant azoïque de formule générale I

$$A-N=N-B(OR)_n \qquad (I)$$

dans laquelle

A est un reste à cinq ou six chaînons, éventuellement benzocyclique, comportant un ou deux hétéro-atomes du groupe N, S et O, ce reste pouvant être substitué une ou plusieurs fois par F, Cl, Br, un groupe alkyle inférieur ayant de 1 à 5 atomes de carbone et/ou par un groupe alcoxy inférieur ayant de 1 à 5 atomes de carbone ou, il est un reste phényle substitué 1 à 3 fois par un groupe alkyle inférieur ayant de 1 à 5 atomes de carbone, un groupe alcoxy inférieur ayant de 1 à 5 atomes de carbone, un groupe nitro, sulfonato et/ou benzoylamino,

B est un reste benzène, naphtalène ou quinoléine pouvant être substitué une ou deux fois par du sulfonato, un groupe alcoxy inférieur ayant de 1 à 5 atomes de carbone, et/ou un groupe alcoxy-poly-éthylène-oxy inférieur, dans lequel le groupe alcoxy possède de 1 à 5 atomes de carbone et peut renfermer de 1 à 5 groupes éthylène-oxy,

R est un reste d'amino-acide ou de di-à pentapeptide pourvu d'un groupe pour la protect ion de l'atome d'azote habituel dans la chimie des peptides, les amino-acides ainsi que les amino-acides du reste peptide étant des L-$\alpha$-amino-acides d'origine naturelle, et,

n est le nombre 1 ou 2,
et le groupe azoïque se trouve en position o ou p par rapport à un des groupes O—R;
la substance tampon réglant avec le liquide à examiner le pH entre 6 et 10 et le chromogène étant présent en une concentration entre $10^{-4}$ et $10^{-1}$ mole/l.

2. Agent de diagnostic selon la revendication 1, caractérisé en ce que le support renferme en outre un agent mouillant et/ou un formateur de complexes.

3. Utilisation d'esters de colorants azoïques de formule générale I

$$A-N=N-B(OR)_n \qquad (I)$$

dans laquelle

A est un reste à cinq ou six chaînons, éventuellement benzocyclique, comportant un ou deux hétéro-atomes du groupe N, S et O, ce reste pouvant être substitué une ou plusieurs fois par F, Cl, Br, un groupe alkyle inférieur ayant de 1 à 5 atomes de carbone et/ou par un groupe alcoxy inférieur ayant 1 à 5 atomes de carbone ou, il est un reste phényle substitué une à trois fois par un groupe alkyle inférieur ayant de 1 à 5 atomes de carbone, un groupe alcoxy inférieur ayant de 1 à 5 atomes de carbone, un groupe nitro, sulfonato et/ou benzoylamino,

B est un reste benzène, naphtalène ou quinoléine pouvant être substitué une ou deux fois par du sulfonato, un groupe alcoxy inférieur ayant de 1 à 5 atomes de carbone, et/ou un groupe alcoxy-poly-éthylène-oxy inférieur, dans lequel le groupe alcoxy possède de 1 à 5 atomes de carbone et peut renfermer de 1 à 5 groupes éthylène-oxy,

R est un reste d'amino-acide ou de di-à pentapeptide pourvu d'un groupe pour la protection de l'atome d'azote habituel dans la chimie des peptides, les amino-acides ainsi que les amino-acides du reste peptide étant des l-$\alpha$-amino-acides d'origine naturelle, et,

n est le nombre 1 ou 2,
et le groupe azoïque se trouve en position o ou p par rapport à un des groupes O—R pour la fabrication d'agents de diagnostic pour la mise en évidence de leucocytes.

4. Esters de colorants azoïques de formule générale I

$$A-N=N-B(OR)_n \quad (I)$$

dans laquelle

A est un reste à cinq ou six chaînons, éventuellement benzocyclique, comportant un ou deux hétéro-atomes du groupe N, S et O, ce reste pouvant être substitué une ou plusieurs fois par F, Cl, Br, un groupe alkyle inférieur ayant de 1 à 5 atomes de carbone et/ou par un groupe alcoxy inférieur ayant de 1 à 5 atomes de carbone ou, il est un reste phényle substitué une à trois fois par un groupe alkyle inférieur ayant de 1 à 5 atomes de carbone, un groupe alcoxy inférieur ayant de 1 à 5 atomes de carbone, un groupe nitro, sulfonato et/ou benzoylamino,
B est un reste benzène, naphthalène ou quinoléine pouvant être substitué une ou deux fois par du sulfonato, un groupe alcoxy inférieur ayant de 1 à 5 atomes de carbone, et/ou un groupe alcoxy-poly-éthylène-oxy inférieur, dans lequel le groupe alcoxy possède de 1 à 5 atomes de carbone et peut renfermer de 1 à 5 groupes éthylène-oxy,
R est un reste d'amino-acide ou de di-à pentapeptide pourvu d'un groupe pour la protection de l'atome d'azote habituel dans la chimie des peptides, les amino-acides ainsi que les amino-acides du reste peptide étant des L-$\alpha$-amino-acides d'origine naturelle, et,

n est le nombre 1 ou 2,
et le groupe azoïque se trouve en position o ou p par rapport à un des groupes O—R.

5. Procédé pour la préparation d'esters de colorants azoïques de formule générale I

$$A-N=N-B(OR)_n \quad (I)$$

dans laquelle

A est un reste à cinq ou six chaînons, éventuellement benzocyclique, comportant un ou deux hétéro-atomes du groupe N, S et O, ce reste pouvant être substitué une ou plusieurs fois par F, Cl, Br, un groupe alkyle inférieur ayant de 1 à 5 atomes de carbone et/ou par un groupe alcoxy inférieur ayant de 1 à 5 atomes de carbone ou, il est un reste phényle substitué une à trois fois par un groupe alkyle, inférieur ayant de 1 à 5 atomes de carbone, un groupe alcoxy inférieur ayant de 1 à 5 atomes de carbone, un groupe nitro, sulfonato et/ou benzoylamino,
B est un reste benzène, naphtalène ou quinoléine pouvant être substitué une ou deux fois par du sulfonato, un groupe alcoxy inférieur ayant de 1 à 5 atomes de carbone, et/ou un groupe alcoxy-poly-éthylène-oxy inférieur, dans lequel le groupe alcoxy possède de 1 à 5 atomes de carbone et peut renfermer de 1 à 5 groupes éthylène-oxy,
R est un reste d'amino-acide ou de di-à pentapeptide pourvu d'un groupe pour la protection de l'atome d'azote habituel dans la chimie des peptides, les amino-acides ainsi que les amino-acides du reste peptide étant des L-$\alpha$-amino-acides d'origine naturelle, et,

n est le nombre 1 ou 2,
et le groupe azoïque se trouve en position o ou p par rapport à un des groupes O—R, caractérisé en ce qu'on fait réagir de façon en soi connue un composé de formule générale II

$$A-N=N-B(OH)_n \quad (II)$$

dans laquelle
A, B et n ont la signification donnée ci-dessus,
avec un acide de formule générale III

$$HO-R \quad (III)$$

dans laquelle
R a la signification donnée ci-dessus,
ou avec un dérivé réactif approprié d'un tel acide.